# EUROPEAN PATENT APPLICATION

(11) **EP 2 769 712 A1**
(43) Date of publication of application: **27.08.2014**
(21) Application number: 13156094.8
(22) Date of filing: 21.02.2013
(51) Int. Cl.: A61K 9/20, A61K 31/4985, A61P 3/04

(54) **Pharmaceutical formulation comprising DPP-IV inhibitor agglomerates and DPP-IV inhibitor particles**

(71) Applicant: Siegfried International AG, 4800 Zofingen (CH)
(72) Inventor: Röhrich, Lambert Tillmann, 4310 Rheinfelden (CH); Paloniemi Legland, Riitta, 2000 Neuchâtel (CH)
(74) Representative: Isarpatent

(57) **Abstract**

The invention relates to a pharmaceutical formulation comprising DPP-IV inhibitor agglomerates and DPP-IV inhibitor particles, wherein the DPP-IV inhibitor is in free form or in form of a salt thereof, wherein at least 45 wt.% of the DPP-IV inhibitor particles and DPP-IV inhibitor agglomerates have a size when measured using sieve analysis of more than 250 µm. The pharmaceutical formulation shows improved stability and allows easy production of solid oral dosage forms due to the good flow properties of the particles and agglomerates.

## Description

The invention relates to a pharmaceutical formulation comprising DPP-IV inhibitor agglomerates and DPP-IV inhibitor particles, wherein the DPP-IV inhibitor is in free form or in form of a salt thereof, wherein at least 45 wt.% of the DPP-IV inhibitor agglomerates and DPP-IV inhibitor particles have a size when measured using sieve analysis of more than 250 µm. The invention further relates to a DPP-IV inhibitor agglomerate comprising a DPP-IV inhibitor in free form or in form of a salt thereof, wherein the DPP-IV inhibitor agglomerate has a size in the range of about 250 µm - about 1000 µm.

### BACKGROUND OF THE INVENTION

The enzyme dipeptidyl peptidase-IV (DPP-IV) is an enzyme associated with immune regulation, signal transduction and apoptosis and degrades polypeptides by cleaving dipeptides from the N-terminals of these polypeptides. In particular, it also plays a major role in glucose metabolism due to the degradation of incretins, such as GLP-1, and low levels of GLP-1 have been related to diseases like type 2 diabetes. Recently research has been ongoing in the field of developing efficient DPP-IV inhibitors that inhibit the DPP-IV enzyme, thus leading to increased levels of GLP-1.

A first compound, (R)-4-oxo-4-[3-(trifluoromethyl)-5,6-dihydro[1,2,4]triazolo[4,3-a]pyrazin-7(8H)-yl]-1-(2,4,5-trifluorophenyl)butan-2-amine, also known as sitagliptin, is disclosed in WO 2003/004498.

A further compound, (S)-1-[N-(3-hydroxy-1-adamantyl(glycyl)]pyrrolidine-2-carbonitrile, also known as vildagliptin, is known from WO 2005/067976.

Further DPP-IV-inhibitor-compounds are, for example, known from WO 2009/085990, WO 2010/000469, WO 2011/018494, and the publication of D. Kim et al., "(2R)-4-oxo-4-[3-(trifluoromethyl)-5,6-dihydro[1,2,4]triazolo[4,3-a]pyrazin-7(8H)-yl]-1-(2,4,5-trifluorophenyl)butan-2-amine: a potent, orally active dipeptidyl peptidase IV inhibitor for the treatment of type-II-diabetes", J. Med. Chem. 2005, 48, 141-151. However, there still remains the need for pharmaceutical formulations comprising a DPP-IV inhibitor with improved stability.

### SUMMARY OF THE INVENTION

The present invention has been made in view of the foregoing, and in particular relates to a pharmaceutical formulation comprising DPP-IV inhibitor agglomerates and DPP-IV inhibitor particles, wherein the DPP-IV inhibitor is in free form or in form of a salt thereof, with improved stability. In particular, at least 45 wt.% of the DPP-IV inhibitor agglomerates and the DPP-IV inhibitor particles in the pharmaceutical formulation have a size when measured using sieve analysis, particularly according to ISO 3310-1, of more than 250 µm.

The invention further relates to DPP-IV inhibitor agglomerates comprising a DPP-IV inhibitor in free form or in form of a salt thereof, wherein the DPP-IV inhibitor agglomerate has a size in the range of about 250 µm to about 1,000 µm.

Further preferred embodiments are referred to in the dependent claims.

### DESCRIPTION OF FIGURES

The invention will now be described with regard to preferred embodiments shown in the Figures. However, the invention is not limited to those preferred embodiments. In particular, the Figures show:
- Fig. 1:: a dissolution profile of film tablets containing 25 mg of sitagliptin phosphate according to comparative example 1;
- Fig. 2:: the dissolution profile of film tablets comprising 100 mg of sitagliptin phosphate according to comparative example 2;
- Fig. 3:: the dissolution profile of tablets comprising 100 mg of sitagliptin fumarate according to example 1;
- Fig. 4:: the dissolution profile of film tablets comprising 25 mg of sitagliptin fumarate according to example 2;
- Fig. 5:: the dissolution profile of film tablets comprising 100 mg of sitagliptin fumarate according to example 3;
- Fig. 6:: dissolution profiles of sitagliptin fumarate cores and film tablets according to comparative examples 1 and 2 and examples 1 - 4;
- Fig. 7:: the dissolution profile of film tablets comprising 25 mg of sitagliptin fumarate according to comparative example 3;

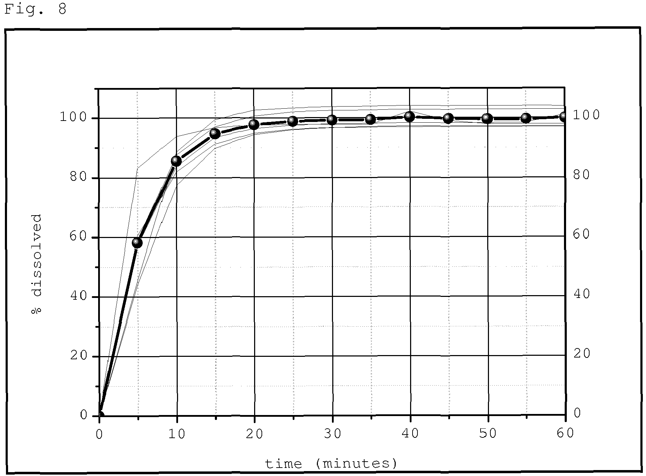

- Fig. 8:: the dissolution profile of film tablets comprising 25 mg of sitagliptin fumarate according to comparative example 4;
- Fig. 9:: dissolution profiles of film tablets according to comparative examples 1, 3 and 4 and example 2;
- Fig. 10:: the result of an X-ray powder diffraction measurement of the DPP-IV inhibitor material used in Reference Example 1

### DETAILED DESCRIPTION OF THE INVENTION

### Definitions:

Any values that are modified by the term "about" in this document generally refer to values that are within an error of two standard deviations from the value according to the applied method, taking into account the measurement conditions and equipment.

All percentages and ratios used therein, unless otherwise noted, are by weight of the total composition, i.e. wt.%, and all measurements have been carried out at ambient temperature and pressure, i.e. 25°C and nominal pressure, unless otherwise noted.

An agglomerate is herein defined to be fused or cemented particles and is thereby defined in contrast to an aggregate, which is a mass of adhered particles, a distinction that also is known from Remington, The Science and Practice of Pharmacy, 22nd Edition, 2012, page 825, which is incorporated herein by reference with regard to the definition of "agglomeration" and "agglomerate". The present agglomerates thus are different from aggregates regarding the binding strength between the particles.

The sizes of particles and agglomerates are determined for the instant invention by sieve analysis, in particular in line with ISO 3310-1. A sieve analysis method is generally known from e.g. Remington, The Science and Practice of Pharmacy, 22nd Edition, 2012, pages 779 - 781, and from European Medicines Agency, ICH Topic Q4B Annex 12, November 2009, available at http://www.ema.europa.eu/docs/en_GB/document_library/Scientifi c_guideline/2010/01/WC500044305.pdf. Using sieving analysis a particle size distribution can be obtained, and the fraction remaining on each sieve, i.e. with a particle size bigger than the mesh size, as well as the fraction at the bottom of the sieving equipment, having passed through all sieves, can be determined by weighing.

By adding the weight of fractions in sieves below a sieve with a certain mesh size, including the bottom, it can be determined which percentage, in wt.%, of these particles compared to all particles in the batch used for sieve analysis is smaller than this mesh size by dividing this sum with the total sum of all fractions and multiplying by 100%. With such an analysis it can be determined whether a majority of the particle size distribution is smaller than a certain mesh size.

Vice versa, a similar approach can also be carried out by adding the weight of all fractions remaining on sieves with a certain mesh size or larger, and dividing this sum by the total weight of all fractions and multiplication by 100%, to determine which percentage in the particle size distribution is larger than this mesh size.

### Description of preferred embodiments:

The present invention relates to a pharmaceutical formulation comprising DPP-IV inhibitor agglomerates and DPP-IV inhibitor particles, wherein the DPP-IV inhibitor is in free form or in form of a salt thereof, wherein at least 45 wt.% of the DPP-IV inhibitor particles and DPP-IV inhibitor agglomerates have a size when measured using sieve analysis, particularly according to ISO 3310-1, of more than 250 µm.

In a particularly preferred embodiment, the sieve analysis of the present invention is carried out with sieves of 125 µm, 250 µm, 500 µm and 800 µm mesh size, preferably in line with ISO 3310-1.

In this respect and in the context of the present invention, a DPP-IV inhibitor also comprises active metabolites and prodrugs thereof, such as active metabolites and prodrugs of DPP-IV inhibitors. A metabolite is an active derivative of a DPP-IV inhibitor produced by a metabolization of the DPP-IV inhibitor. A prodrug is a compound that is metabolized to a DPP-IV inhibitor or is metabolized to the same metabolite as a DPP-IV inhibitor.

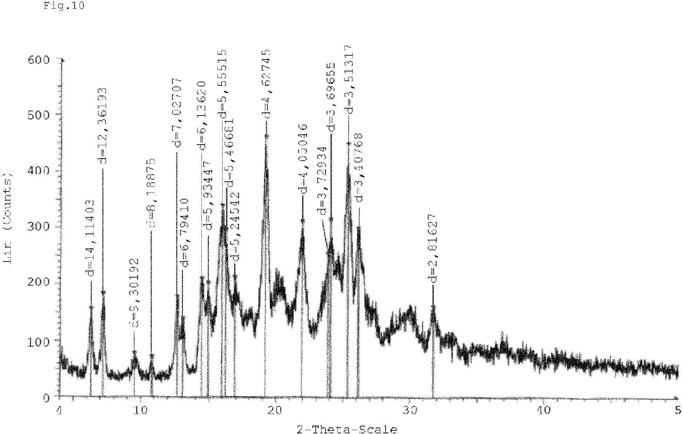

DPP-IV inhibitors that can be used in the present invention are known in the art, for example from WO 2003/004498, WO 2005/067976, WO 2009/085990, WO 2010/000469 and WO 2011/018494, which are herein referred to in regard to DPP-IV inhibitors. In a preferred embodiment, the DPP-IV inhibitor is (R)-4-oxo-4-[3-(trifluoromethyl)-5,6-dihydro[1,2,4]triazolo[4,3-a]pyrazin-7(8H)-yl]-1-(2,4,5-trifluorophenyl)butan-2-amine (sitagliptin) or a salt thereof. A particularly preferred DPP-IV inhibitor is an acid salt of sitagliptin, and particularly preferably a fumaric acid salt, i.e. a fumarate, of sitagliptin is used. In a further preferred embodiment, the DPP-IV inhibitor is a polymorph of the fumaric acid salt of (R)-4-oxo-4-[3-(trifluoromethyl)-5,6-dihydro[1,2,4]triazolo[4,3-a]pyrazin-7(8H)-yl]-1-(2,4,5-trifluorophenyl)butan-2-amine which exhibits a characteristic X-ray powder diffraction pattern substantially equal to the one in Figure 10 when measured using Kα-radiation of a copper anode. In preferred embodiments, the copper anode has a wavelength of 1.541838 Å. Preferably the polymorph of the fumaric acid salt of (R)-4-oxo-4-[3-(trifluoromethyl)-5,6-dihydro[1,2,4]triazolo[4,3-a]pyrazin-7(8H)-yl]-1-(2,4,5-trifluorophenyl)butan-2-amine has peaks at the following 2-theta-values when measured using Kα-radiation of a copper anode: 6,257; 7,145; 9,500; 10,795; 12,586; 13,020; 14,423; 14,916; 15,941; 16,200; 16,889; 19,164; 21,926; 23,840; 24,055; 25,331; 26,128; 31,747. Further preferably the polymorph of the fumaric acid salt of (R)-4-oxo-4-[3-(trifluoromethyl)-5,6-dihydro[1,2,4]triazolo[4,3-a]pyrazin-7(8H)-yl]-1-(2,4,5-trifluorophenyl)butan-2-amine exhibits a characteristic X-ray powder diffraction pattern with the following characteristic peaks of the following 2-theta values when measured using Kα-radiation of a copper anode:

| 2-theta | I (counts) |
|---|---|
| 6,257 | 153; |
| 7,145 | 180; |
| 9,500 | 74; |
| 10, 795 | 67; |
| 12, 586 | 174; |
| 13, 020 | 136; |
| 14, 423 | 206; |
| 14, 916 | 199; |
| 15, 941 | 337; |
| 16, 200 | 294; |
| 16, 889 | 209; |
| 19, 164 | 459; |
| 21, 926 | 309; |
| 23, 840 | 251; |
| 24, 055 | 312; |
| 25, 331 | 447; |
| 26, 128 | 297; |
| 31, 747 | 157. |

In a preferred embodiment, at least 50 wt.% of the DPP-IV inhibitor particles and DPP-IV inhibitor agglomerates have a size when measured using sieve analysis, particularly according to ISO 3310-1, of more than 250 µm, and in a particularly preferred embodiment at least 55 wt.% of the DPP-IV inhibitor particles and DPP-IV inhibitor agglomerates have a size when measured using sieve analysis, particularly according to ISO 3310-1, of more than 250 µm. Further preferably at least 60 wt.% of the DPP-IV inhibitor particles and DPP-IV inhibitor agglomerates have a size when measured using sieve analysis, particularly according to ISO 3310-1, of more than 250 µm, and even further preferably at least 65 wt.%.

In a further preferred embodiment, at least 10 wt.% of the DPP-IV inhibitor particles and DPP-IV inhibitor agglomerates have a size when measured using sieve analysis, particularly according to ISO 3310-1, of more than 500 µm, and more preferably at least 15 wt.% and even more preferably 20 wt.% of the DPP-IV inhibitor particles and DPP-IV inhibitor agglomerates have a size when measured using sieve analysis, particularly according to ISO 3310-1, of more than 500 µm.

Thus, in particularly preferred embodiments, a pharmaceutical formulation according to the invention comprises DPP-IV inhibitor agglomerates and DPP-IV inhibitor particles, wherein the DPP-IV inhibitor is the fumaric acid salt of (R)-4-oxo-4-[3-(trifluoromethyl)-5,6-dihydro[1,2,4]triazolo[4,3-a]pyrazin-7(8H)-yl]-1-(2,4,5-trifluorophenyl)butan-2-amine (sitagliptin), and wherein at least 45 wt.% of the DPP-IV inhibitor particles and DPP-IV inhibitor agglomerates have a size when measured using sieve analysis, particularly according to ISO 3310-1, of more than 250 µm, more preferably at least 50 wt.%, even more preferably at least 55 wt.%, further preferably at least 60 wt.% and even further preferably at least 60 wt.%.

In a further particularly preferred embodiment, a pharmaceutical formulation according to the invention comprises DPP-IV inhibitor agglomerates and DPP-IV inhibitor particles, wherein the DPP-IV inhibitor is the fumaric acid salt of (R)-4-oxo-4-[3-(trifluoromethyl)-5,6-dihydro[1,2,4]triazolo[4,3-a]pyrazin-7(8H)-yl]-1-(2,4,5-trifluorophenyl)butan-2-amine (sitagliptin), and wherein at least 10 wt.% of the DPP-IV inhibitor particles and DPP-IV inhibitor agglomerates have a size when measured using sieve analysis, particularly according to ISO 3310-1, of more than 500 µm, and more preferably at least 15 wt.% and even more preferably 20 wt.% of the DPP-IV inhibitor particles and DPP-IV inhibitor agglomerates have a size when measured using sieve analysis, particularly according to ISO 3310-1, of more than 500 µm.

Furthermore described in an embodiment of the invention is a pharmaceutical formulation comprising DPP-IV inhibitor particles, wherein the DPP-IV inhibitor is the fumaric acid salt of (R)-4-oxo-4-[3-(trifluoromethyl)-5,6-dihydro[1,2,4]triazolo[4,3-a]pyrazin-7(8H)-yl]-1-(2,4,5-trifluorophenyl)butan-2-amine (sitagliptin), and wherein at least 45 wt.% of the DPP-IV inhibitor particles have a size when measured using sieve analysis, particularly according to ISO 3310-1, of more than 250 µm, more preferably at least 50 wt.%, even more preferably at least 55 wt.%, further preferably at least 60 wt.% and even further preferably at least 60 wt.%. Further preferably the pharmaceutical formulation comprises DPP-IV inhibitor particles, wherein the DPP-IV inhibitor is the fumaric acid salt of (R)-4-oxo-4-[3-(trifluoromethyl)-5,6-dihydro[1,2,4]triazolo[4,3-a]pyrazin-7(8H)-yl]-1-(2,4,5-trifluorophenyl)butan-2-amine (sitagliptin), and wherein at least 10 wt.% of the DPP-IV inhibitor particles have a size when measured using sieve analysis, particularly according to ISO 3310-1, of more than 500 µm, and more preferably at least 15 wt.% and even more preferably 20 wt.% of the DPP-IV inhibitor particles have a size when measured using sieve analysis, particularly according to ISO 3310-1, of more than 500 µm.

Particularly preferred is a pharmaceutical composition comprising DPP-IV inhibitor particles wherein the DPP-IV inhibitor is a polymorph of the fumaric acid salt of (R)-4-oxo-4-[3-(trifluoromethyl)-5,6-dihydro[1,2,4]triazolo[4,3-a]pyrazin-7(8H)-yl]-1-(2,4,5-trifluorophenyl)butan-2-amine which exhibits a characteristic X-ray powder diffraction pattern substantially equal to the one in Figure 10 when measured using Kα-radiation of a copper anode.

In certain embodiments, the pharmaceutical formulation of the invention further comprises one or more pharmaceutically acceptable excipients.

These excipients are well-known to the skilled person, e.g. from Remington, The Science and Practice of Pharmacy, 22nd Edition, 2012, which is incorporated herein by reference in regard to pharmaceutical excipients, particularly volume 1: "The Science of Pharmacy", pages 1049-1070.

Excipients that can be used in the pharmaceutical formulation of the present invention include, but are not limited to:
- Antioxidants like butylated hydroxyanisole and butylated hydroxytoluene
- binders like alginic acid, sodium alginate, starch and derivatives thereof, cellulose and derivatives thereof, carboxymethyl cellulose sodium (CMC), microcrystalline cellulose (MCC), dextrin, dextrose, guar gum, lactose, povidone ,copovidone, tragacanth, zein
- coating agents like carboxymethyl cellulose sodium (CMC), carnauba wax, cellulose acetate phthalate, gelatin, hydroxypropyl cellulose (HPC),hydroxypropyl methylcellulose (HPMC), polyvinyl alcohol (PVA), poly(meth)acrylates, maltodextrin, methylcellulose, starch, sucrose or commercially coating agents, as used in the examples,
- colors and pigments,
- diluents/fillers like microcrystalline cellulose, calcium hydrogen phosphate, calcium carbonate, calcium sulfate, microcrystalline cellulose (MCC), powdered cellulose, dextrates, dextrose, dextrin, kaolin, lactose, maltodextrin, mannitol, starch, sucrose
- disintegrants like croscarmellose sodium, crospovidone, guar gum, sodium starch glycolate, L-HPC (low-substituted hydroxypropyl cellulose)
- emollients like glycerin, glyceryl monostearate, isopropyl myristate, polyethylene glycols
- emulsifiers like carbomer, carrageenan, lanolin, lecithin, mineral oil, pectin, sorbitan esters
- flavors
- glidants/antiadherents like talc or colloidal silicon dioxide
- humectants like glycerin or sorbitol
- lubricants like calcium stearate, magnesium stearate, sodium stearyl fumarate, stearic acid, talc, polyethylene glycol (PEG)
- plasticizers like glycerin, triethyl citrate, triacetin, di-butyl phtalate
- preservatives like alcohol, parabens or sorbic acid
- surfactants like polyethylene glycols, polysorbates, sodium lauryl sulphates
- suspending agents like acacia, agar, kaolin, pectin
- sweeteners like sorbitol, sucrose, dextrose, fructose, mannitol, xylitol, aspartame

Also further excipients can be used. Suitable excipients are also known from e.g. WO 2005/067976, which is incorporated hereby in regard to excipients.

In certain embodiments, the pharmaceutical formulation of the present invention can further comprise a second or furtherpharmaceutically active ingredients.

Particularly suited second pharmaceutically active ingredients comprise biguanides such as metformin. Metformin hydrochloride is particularly preferred.

In certain embodiments of the present invention, the pharmaceutical formulation comprising DPP-IV inhibitor agglomerates and DPP-IV inhibitor particles further comprise a sulfonylurea selected from the group consisting of tolbutamide, acetohexamide, tolazamide, chlorpropamide, glipizide, glyburide, glimepiride and gliclazide, preferably glimepiride.

In certain embodiments of the present invention, the pharmaceutical formulation comprising DPP-IV inhibitor agglomerates and DPP-IV inhibitor particles further comprise a peroxisome proliferator-activated receptor gamma (PPARγ) agonist selected from the group consisting of rosiglitazone, pioglitazone and troglitazone, preferably pioglitazone.

In certain embodiments of the present invention, the pharmaceutical formulation comprising DPP-IV inhibitor agglomerates and DPP-IV inhibitor particles further comprise a 3-hydroxy-3-methyl-glutaryl-coenzyme A reductase (HMG-CoA reductase) inhibitor selected from the group consisting of lovastatin, simvastatin, pravastatin, fluvastatin, atorvastatin, rivastatin, itavastatin and rosuvastatin, preferably simvastatin.

Furthermore the pharmaceutical formulation of the present invention can in certain embodiments be coated with suitable coating substances, and can preferably be film-coated. Suitable coatings and coating methods are known to the skilled person, for example from Remington, The Science and Practice of Pharmacy, 22nd Edition, 2012, which is incorporated herein by reference in regard to pharmaceutical excipients, particularly volume 1: "The Science of Pharmacy", pages 947-987. The coating amount can be suitably set by the skilled person based on the purpose of the coating.

The amounts of the DPP-IV inhibitor agglomerates and DPP-IV inhibitor particles, one or more of the pharmaceutically acceptable excipient(s) and the optional second or further pharmaceutically active ingredient(s) in the pharmaceutical formulation of the present invention are not particularly limited and can be suitably set by a skilled person based on his general knowledge and the state of the art. Furthermore also a medical practitioner will be able to suitably set individual amounts for administration to different subjects based on each subject's needs and constitution.

In certain embodiments, the pharmaceutical formulation of the present invention can comprise 1 - 80 wt.% of the DPP-IV inhibitor agglomerates and DPP-IV inhibitor particles, up to 99 wt.% of the excipients and 0 to 80 wt.% of the second or further pharmaceutically active ingredient. The pharmaceutical formulation further can be optionally coated.

In certain embodiments the ranges (in wt.%) used in the pharmaceutical formulation according to the invention are as follows:
DPP-IV inhibitor agglomerates and DPP-IV inhibitor particles: up to 60%;
at least one filler/diluent: up to 95%;
at least one disintegrant: up to 20%;
at least one glidant/lubricant: up to 20%;
optionally about 0 to 80 wt.% of the second or further pharmaceutically active ingredient; and
optionally a film coat.

In preferable embodiments the pharmaceutical formulation of the present invention may comprise about 10-50 wt.% of the DPP-IV inhibitor agglomerates and DPP-IV inhibitor particles, about 40-80 wt.% of at least one diluent/filler, about 0-10 wt.% of at least one disintegrant, about 0-10 wt.% of at least one glidant/lubricant, optionally about 0 to 80 wt.% of the second or further pharmaceutically active ingredient and optionally a film coat.

The further preferable ranges (in wt.%) used in the pharmaceutical formulation according to the present invention are as follows:
DPP-IV inhibitor agglomerates and DPP-IV inhibitor particles: about 10-50%;
at least one filler/diluent: about 50-80%;
at least one disintegrant: about 0.1-10%;
at least one glidant/lubricant: about 0.1-10%;
optionally about 0 to 80 wt.% of the second or further pharmaceutically active ingredient; and
optionally a film coat.

The most preferable ranges (in wt.%) used in the pharmaceutical formulation according to the present invention are as follows:
DPP-IV inhibitor agglomerates and DPP-IV inhibitor particles: about 20-40 %;
Filler/diluent: about 60-70 %;
Disintegrant: about 1-6 %;
Glidant/lubricant: about 1-6 %;
optionally about 0 to 80 wt.% of the second or further pharmaceutically active ingredient; and
optionally a film coat.

It is also possible to administer the present pharmaceutical formulation together with a separate second pharmaceutical formulation comprising a different active ingredient, e.g. the above second pharmaceutically active ingredient, which can be suitably determined by a skilled person, e.g. a medical practitioner, based on the subject's needs.

The pharmaceutical formulations of the present invention include formulations suitable for being administered orally, subcutaneously, parenterally, locally (ointments, creams, powders), in the form of droplets, as a nasal or mouth spray.

The pharmaceutical formulation of the present invention is preferably provided in the form of an oral dosage form. The oral dosage form can be thereby a solid oral dosage form, like powders, granules, pellets, tablets and capsules, or a liquid oral dosage form, like syrups, dispersions and emulsions. Preferably, the pharmaceutical formulation is provided in the form of a solid oral dosage form. Examples of solid oral dosage forms are known to the skilled person from e.g. Remington, The Science and Practice of Pharmacy, 2nd edition, 2012, volume 1: The Science of Pharmacy, pages 947-976.

Methods of preparing the solid oral dosage forms are known to the skilled person. Preferable methods for preparing tablets are direct compression and dry and wet granulation (e.g. high shear or fluid bed). Direct compression is particularly preferred.

Preferably the solid dosage form is in the form of a tablet, coated tablet, capsule, pill, powder or granule. The formulations can be adapted for immediate release, delayed release or modified release of the active ingredient.

It is possible that the solid oral dosage form is uncoated or coated, e.g. film coated, powder coated, enteric coated, sugar coated or modified release coated. Suitable substances for coating are known to the skilled person.

Preferred as solid oral dosage form are tablets and coated tablets as the pharmaceutical formulation of the present invention can be easily subjected to direct compression. In this regard, a pharmaceutical composition comprising DPP-IV inhibitor agglomerates and DPP-IV inhibitor particles wherein at least 45 wt.% of the DPP-IV inhibitor agglomerates and DPP-IV inhibitor particles have a size when measured using sieve analysis of more than 250 µm leads to improved flow properties, which in turn lead to improved compressibility and quick disintegration of the solid oral dosage form after direct compression. Improved flow properties, which in turn lead to improved compressibility and quick disintegration of a solid oral dosage form after direct compression results can also be obtained with a pharmaceutical formulation comprising DPP-IV inhibitor particles, wherein the DPP-IV inhibitor is the fumaric acid salt of (R)-4-oxo-4-[3-(trifluoromethyl)-5,6-dihydro[1,2,4]triazolo[4,3-a]pyrazin-7(8H)-yl]-1-(2,4,5-trifluorophenyl)butan-2-amine (sitagliptin), and wherein at least 45 wt.% of the DPP-IV inhibitor particles have a size when measured using sieve analysis, particularly according to ISO 3310-1, of more than 250 µm.

The present invention further relates to DPP-IV inhibitor agglomerates comprising a DPP-IV inhibitor in free form or in form of a salt thereof, wherein the DPP-IV inhibitor agglomerate has a size in the range of about 250 µm to about 1,000 µm, preferably about 250 µm to about 750 µm, further preferably about 275 µm to about 600 µm, even further preferably from about 300 µm to about 600 µm, or preferably from 250 µm to about 500 µm, further preferably from about 300 µm to about 500 µm.

Preferably the present invention also relates to DPP-IV inhibitor agglomerates comprising a DPP-IV inhibitor in free form or in form of a salt thereof, wherein the agglomerate has a particle size in the range of 250 µm to 1,000 µm, preferably 250 µm to 750 µm, further preferably 275 µm to 600 µm, even further preferably 300 µm to 600 µm, or preferably 250 µm to 500 µm, further preferably 300 µm to 500 µm.

In preferable embodiments, the DPP-IV inhibitor in the present DPP-IV inhibitor agglomerate is sitagliptin or a salt thereof, preferably the fumaric acid salt of sitagliptin, and particularly preferred a polymorph of the fumaric acid salt of (R)-4-oxo-4-[3-(trifluoromethyl)-5,6-dihydro[1,2,4]triazolo[4,3-a]pyrazin-7(8H)-yl]-1-(2,4,5-trifluorophenyl)butan-2-amine which exhibits a characteristic X-ray powder diffraction pattern substantially equal to the one in Figure 10 when measured using Kα-radiation of a copper anode. In preferred embodiments, the copper anode has a wavelength of 1.541838 Å. Preferably the polymorph of the fumaric acid salt of (R)-4-oxo-4-[3-(trifluoromethyl)-5,6-dihydro[1,2,4]triazolo[4,3-a]pyrazin-7(8H)-yl]-1-(2,4,5-trifluorophenyl)butan-2-amine has peaks at the following 2-theta-values when measured using Kα-radiation of a copper anode: 6,257; 7,145; 9,500; 10,795; 12,586; 13,020; 14,423; 14,916; 15,941; 16,200; 16,889; 19,164; 21,926; 23,840; 24,055; 25,331; 26,128; 31,747. Further preferably the polymorph of the fumaric acid salt of (R)-4-oxo-4-[3-(trifluoromethyl)-5,6-dihydro[1,2,4]triazolo[4,3-a]pyrazin-7(8H)-yl]-1-(2,4,5-trifluorophenyl)butan-2-amine exhibits a characteristic X-ray powder diffraction pattern with the following characteristic peaks of the following 2-theta values when measured using Kα-radiation of a copper anode:

| 2-theta | I (counts) |
|---|---|
| 6, 257 | 153; |
| 7, 145 | 180; |
| 9, 500 | 74; |
| 10, 795 | 67; |
| 12, 586 | 174; |
| 13, 020 | 136; |
| 14, 423 | 206; |
| 14, 916 | 199; |
| 15, 941 | 337; |
| 16, 200 | 294; |
| 16, 889 | 209; |
| 19, 164 | 459; |
| 21, 926 | 309; |
| 23, 840 | 251; |
| 24, 055 | 312; |
| 25, 331 | 447; |
| 26, 128 | 297; |
| 31, 747 | 157. |

A preferred DPP-IV inhibitor agglomerate of the present invention thus comprises sitagliptin fumarate as DPP-IV inhibitor and has a particle size in the range of about 250 µm to about 1,000 µm, preferably of about 250 µm to about 750 µm, further preferably of about 275 µm to about 600 µm, even further preferably from about 300 µm to about 600 µm, or from about 250 µm to about 500 µm, preferably from about 300 µm to about 500 µm.

A further preferred DPP-IV inhibitor agglomerate of the present invention comprises sitagliptin fumarate as DPP-IV inhibitor and has a particle size in the range of 250 µm to 1,000 µm, preferably of 250 µm to 750 µm, further preferably of 275 µm to 600 µm, even further preferably from 300 µm to 600 µm, or from 250 µm to 500 µm, preferably from 300 µm to 500 µm.

In a further embodiment, the present invention also relates to DPP-IV inhibitor particles comprising a DPP-IV inhibitor in free form or in form of a salt thereof, wherein the particle has a particle size in the range of 250 µm to 1,000 µm, preferably 250 µm to 750 µm, further preferably 275 µm to 600 µm, even further preferably 300 µm to 600 µm, or preferably 250 µm to 500 µm, further preferably 300 µm to 500 µm. Further, the present invention also relates to DPP-IV inhibitor particles wherein the DPP-IV inhibitor is a polymorph of the fumaric acid salt of (R)-4-oxo-4-[3-(trifluoromethyl)-5,6-dihydro[1,2,4]triazolo[4,3-a]pyrazin-7(8H)-yl]-1-(2,4,5-trifluorophenyl)butan-2-amine which exhibits a characteristic X-ray powder diffraction pattern substantially equal to the one in Figure 10 when measured using Kα-radiation of a copper anode. In preferred embodiments, the copper anode has a wavelength of 1.541838 Å. Preferably the polymorph of the fumaric acid salt of (R)-4-oxo-4-[3-(trifluoromethyl)-5,6-dihydro[1,2,4]triazolo[4,3-a]pyrazin-7(8H)-yl]-1-(2,4,5-trifluorophenyl)butan-2-amine has peaks at the following 2-theta-values when measured using Kα-radiation of a copper anode: 6,257; 7,145; 9,500; 10,795; 12,586; 13,020; 14,423; 14,916; 15,941; 16,200; 16,889; 19,164; 21,926; 23,840; 24,055; 25,331; 26,128; 31,747. Further preferably the polymorph of the fumaric acid salt of (R)-4-oxo-4-[3-(trifluoromethyl)-5,6-dihydro[1,2,4]triazolo[4,3-a]pyrazin-7(8H)-yl]-1-(2,4,5-trifluorophenyl)butan-2-amine exhibits a characteristic X-ray powder diffraction pattern with the following characteristic peaks of the following 2-theta values when measured using Kα-radiation of a copper anode:

| 2-theta | I (counts) |
|---|---|
| 6, 257 | 153; |
| 7, 145 | 180; |
| 9, 500 | 74; |
| 10, 795 | 67; |
| 12, 586 | 174; |
| 13, 020 | 136; |
| 14, 423 | 206; |
| 14, 916 | 199; |
| 15, 941 | 337; |
| 16, 200 | 294; |
| 16, 889 | 209; |
| 19, 164 | 459; |
| 21, 926 | 309; |
| 23, 840 | 251; |
| 24, 055 | 312; |
| 25, 331 | 447; |
| 26, 128 | 297; |
| 31, 747 | 157. |

In preferred embodiments, the DPP-IV inhibitor particles of the present invention, which can be used in the present pharmaceutical formulation, are obtained, at least in part, as agglomerates.

These agglomerates have the advantage that they can be used as they are and do not have to be subjected to further processing, like milling. The DPP-IV inhibitor is thus not negatively influenced by such further processing, which further processing could lead to a modification of the substance. Apart from that, it is economically more effective not to subject the DPP-IV inhibitor agglomerates and/or particles to further processes, which thus also leads to a reduction of cost. Surprisingly it has been found that the present pharmaceutical formulation comprising DPP-IV inhibitor agglomerates and DPP-IV inhibitor particles, wherein the DPP-IV inhibitor is in free form or in form of a salt thereof, wherein at least 45 wt.% of the DPP-IV inhibitor agglomerates and DPP-IV inhibitor particles have a size when measured using sieve analysis of more than 250 µm can be obtained without further processing of the DPP-IV inhibitor particles, leading to improved flow properties and easy compressibility. Tablets obtained by compression of the present pharmaceutical formulation show improved compressibility and a quick disintegration time. This means that for a certain target hardness of the tablet a reduced pressure is required during fabrication compared to pharmaceutical formulations wherein not at least 45 wt.% of the DPP-IV inhibitor agglomerates and DPP-IV inhibitor particles have a size when measured using sieve analysis of more than 250 µm.

In the preferred embodiments of the present pharmaceutical formulation or of the present DPP-IV inhibitor particle, comprising sitagliptin fumarate, i.e. the fumaric acid salt of sitagliptin, as DPP-IV inhibitor, the ratio of sitagliptin to fumaric acid is not particularly limited. A preferred ratio of sitagliptin to fumaric acid is in the weight range of 1:0.5 to 1:1, preferably in the range of 1:0.6 to 1:0.9, further preferably in the range of 1:0.7 to 1:0.8, and particularly preferably in the range of 1:0.75.

Another aspect of the present invention is the use of the pharmaceutical formulation comprising DPP-IV inhibitor agglomerates and DPP-IV inhibitor particles and/or the DDP-IV inhibitor agglomerates and/or the DPP-IV inhibitor particles of the present invention for the manufacture of a medicament.

In certain embodiments of the present invention, the use of the pharmaceutical formulation comprising DPP-IV inhibitor agglomerates and DPP-IV inhibitor particles and/or the DDP-IV inhibitor agglomerates and/or the DPP-IV inhibitor particles of the present invention is for the manufacture of a medicament to improve blood glucose control in type 2 diabetes mellitus patients.

Another aspect of the present invention is the use of the pharmaceutical formulation comprising DPP-IV inhibitor agglomerates and DPP-IV inhibitor particles and/or the DDP-IV inhibitor agglomerates and/or the DPP-IV inhibitor particles of the present invention in combination with a biguanide, preferably metformin, particularly preferably metformin hydrochloride, for the manufacture of a medicament to improve blood glucose control in type 2 diabetes mellitus patients.

In certain embodiments of the present invention, the use of the pharmaceutical formulation comprising DPP-IV inhibitor agglomerates and DPP-IV inhibitor particles and/or the DDP-IV inhibitor agglomerates and/or the DPP-IV inhibitor particles of the present invention is in combination with a sulfonylurea selected from the group consisting of tolbutamide, acetohexamide, tolazamide, chlorpropamide, glipizide, glyburide, glimepiride and gliclazide, preferably glimepiride, for the manufacture of a medicament to improve blood glucose control in type 2 diabetes mellitus patients.

In certain embodiments of the present invention, the use of pharmaceutical formulation comprising DPP-IV inhibitor agglomerates and DPP-IV inhibitor particles and/or the DDP-IV inhibitor agglomerates and/or the DPP-IV inhibitor particles of the present invention is in combination with a peroxisome proliferator-activated receptor gamma (PPARγ) agonist selected from the group consisting of rosiglitazone, pioglitazone and troglitazone, preferably pioglitazone, for the manufacture of a medicament to improve blood glucose control in type 2 diabetes mellitus patients.

In certain embodiments of the present invention, the use of the pharmaceutical formulation comprising DPP-IV inhibitor agglomerates and DPP-IV inhibitor particles and/or the DDP-IV inhibitor agglomerates and/or the DPP-IV inhibitor particles of the present invention is in combination with a 3-hydroxy-3-methyl-glutaryl-coenzyme A reductase (HMG-CoA reductase) inhibitor selected from the group consisting of lovastatin, simvastatin, pravastatin, fluvastatin, atorvastatin, rivastatin, itavastatin and rosuvastatin, preferably simvastatin, for the manufacture of a medicament to improve blood glucose control in type 2 diabetes mellitus patients.

While the present invention has been described with reference to preferred embodiments thereof, it will now be described in more detail with reference to the following examples, which, however, do not limit the scope of the invention.

### EXAMPLES

### Comparative examples 1 and 2

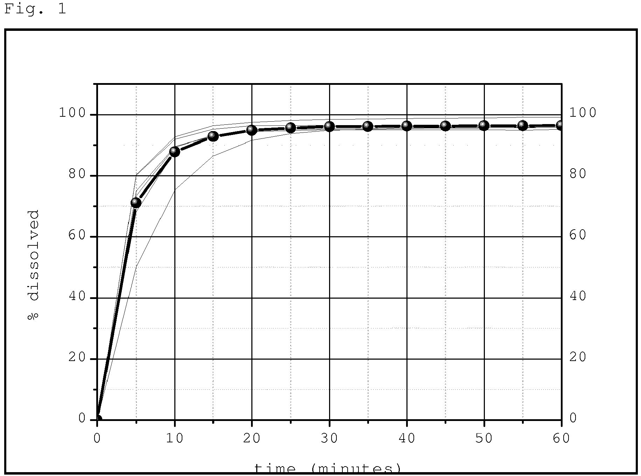

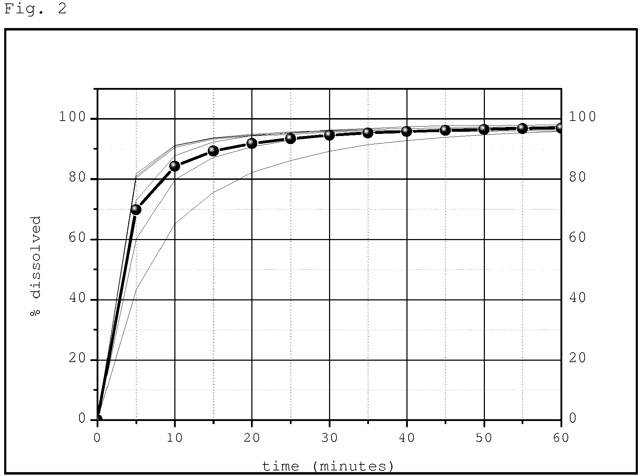

Six tablets containing 25 mg (comparative example 1; batch no. W21888) and 100 mg (comparative example 2; batch no. Y0086) of sitagliptin phosphate (Januvia, Merck & Co) with the compositions given in Table 1 (composition taken from sweweb.mpa.se/swedisii) were subjected to a dissolution test in 900 ml 0.1 N HCl at a stirring rate of 50 rounds/minute. The results are shown in tables 2 and 3 and Figs. 1 and 2.

**Table 1: Composition of tablets of comparative examples 1, 2**

| Comparative example | 1 | 2 |
|---|---|---|
| Dosage strength and tablet type | 25 mg FCT | 100 mg FCT |
| Sitagliptin phopshpate | 32.13 | 128.5 |
| Calcium hydrogen phosphate anhydrous (direct compression grade) | 30.94 | 123.8 |
| Avicel 102 (microcrystalline cellulose) | 30.94 | 123.8 |
| AcDiSol (croscarmellose sodium) | 2.0 | 8.0 |
| Sodiumstearylfumarate (PRUV) | 3.0 | 12.0 |
| Magnesiumstearate | 1.0 | 4.0 |
| Opadry II (Colorcon) | 4.0 | 16.0 |
| Sum | 104 | 416 |

| | | |
|---|---|---|
| Amounts in Table 1 are given in mg. | | |

**Table 2: Dissolution results of tablets comprising 25 mg of sitagliptin phosphate (Januvia, at Paddle 50rpm, 900ml 0.1 N HCl) of comparative example 1**

| **Dissolution** | | | | | | | |
|---|---|---|---|---|---|---|---|
| **Time (min.)** | **Tbl. 1 (%)** | **Tbl. 2 (%)** | **Tbl. 3 (%)** | **Tbl. 4 (%)** | **Tbl. 5 (%)** | **Tbl. 6 (%)** | **Mean (%)** |
| 0 | 0.0 | 0.0 | 0.0 | 0.0 | 0.0 | 0.0 | **0.0** |
| 5 | 82.6 | 75.6 | 82.7 | 51.6 | 70.2 | 77.2 | **73.3** |
| 10 | 94.7 | 92.1 | 95.6 | 77.6 | 90.7 | 92.4 | **90.5** |
| 15 | 98.2 | 96.2 | 99.3 | 89.1 | 95.7 | 95.8 | **95.7** |
| 20 | 99.2 | 97.4 | 100.4 | 94.3 | 97.9 | 97.3 | **97.8** |
| 25 | 99.3 | 98.0 | 101.1 | 96.6 | 98.6 | 97.7 | **98.5** |
| 30 | 99.4 | 98.3 | 101.5 | 97.7 | 98.9 | 97.9 | **99.0** |
| 35 | 99.4 | 98.4 | 101.5 | 98.1 | 98.9 | 97.9 | **99.0** |
| 40 | 99.4 | 98.6 | 101.8 | 98.4 | 99.0 | 98.0 | **99.2** |
| 45 | 99.3 | 98.6 | 101.9 | 98.5 | 98.9 | 97.9 | **99.2** |
| 50 | 99.4 | 98.6 | 101.9 | 98.6 | 99.0 | 97.9 | **99.2** |
| 55 | 99.4 | 98.7 | 102.1 | 98.8 | 99.1 | 97.8 | **99.3** |
| 60 | 99.5 | 98.8 | 102.3 | 98.8 | 99.1 | 98.0 | **99.4** |

**Table 3: Dissolution results of tablets comprising 100 mg of sitagliptin phosphate (Januvia, at Paddle 50rpm, 900ml 0.1 N HCl) of comparative example 2**

| **Dissolution** | | | | | | | |
|---|---|---|---|---|---|---|---|
| **Time (min.)** | **Tbl. 1 (%)** | **Tbl. 2 (%)** | **Tbl. 3 (%)** | **Tbl. 4 (%)** | **Tbl. 5 (%)** | **Tbl. 6 (%)** | **Mean (%)** |
| 0 | 0.0 | 0.0 | 0.0 | 0.0 | 0.0 | 0.0 | **0.0** |
| 5 | 43.1 | 80.8 | 73.0 | 60.0 | 81.7 | 80.3 | **69.8** |
| 10 | 65.1 | 91.0 | 87.7 | 79.7 | 91.2 | 90.5 | **84.2** |
| 15 | 75.6 | 93.6 | 92.3 | 87.2 | 93.7 | 93.3 | **89.3** |
| 20 | 82.1 | 94.6 | 94.4 | 90.7 | 94.9 | 94.3 | **91.8** |
| 25 | 86.1 | 95.3 | 95.4 | 93.0 | 95.7 | 94.8 | **93.4** |
| 30 | 89.2 | 96.0 | 96.3 | 94.2 | 96.1 | 95.6 | **94.6** |
| 35 | 91.4 | 96.2 | 96.8 | 95.3 | 96.5 | 95.7 | **95.3** |
| 40 | 92.8 | 96.2 | 97.4 | 96.0 | 96.8 | 95.7 | **95.8** |
| 45 | 93.9 | 96.6 | 97.7 | 96.6 | 97.0 | 95.7 | **96.2** |
| 50 | 94.7 | 96.5 | 97.7 | 96.9 | 97.2 | 95.9 | **96.5** |
| 55 | 95.4 | 96.8 | 97.9 | 97.3 | 97.3 | 96.1 | **96.8** |
| 60 | 95.8 | 96.8 | 98.0 | 97.6 | 97.4 | 96.0 | **96.9** |

### Reference Example 1

A batch of sitagliptin fumarate particles were measured using sieve analysis. A test sieve rack with sieves of the company Retsch (Retsch Test Sieve 200 mm diameter x 25 mm height), conforming to ISO 3310-1, with mesh sizes of 125 µm, 250 µm, 500 µm and 800 µm were applied for the sieve analysis using standard procedure for sieve analysis in line with ISO 3310-1.

The results of the sieve analysis are as follows:

| | |
|---|---|
| Bottom | 14.0 wt.% |
| 125 µm | 26.49 wt.% |
| 250 **µ**m | 33.85 wt.% |
| 500 **µ**m | 21.94 wt.% |
| 800 **µ**m | 3.2 wt.% |

| | |
|---|---|
| Sum 58.99 wt.% > 250 **µ**m | |

It was further confirmed by optical microscopy that the larger particles with sizes over 250 µm were present at least partially as agglomerates.

Further, an X-ray powder diffraction (XRPD) measurement was carried out on a sample of this batch using a D5000 Siemens X-ray diffractometer having a graphite secondary monochromator and a scintillation detector at an ambient temperature kept at 21°C. Spectra were obtained using the Kα radiation of a copper anode with a wavelength of 1.541838 Å, a voltage V of 50 kV and a current I of 20 mA. The measurement started at 4.000° on the 2-Theta-Scale and was carried out till 50.000°at a stepsize of 0.02° and a step time of 1s. The results of this measurement are shown in Fig. 10. The 2-Theta-values, lattice plane distances d(A) and their respective intensities (in counts) are given in Figure 10 and the following table 4. Spectra were obtained with the following settings: wavelength 1 1.54056 Å, wavelength 2 1,54439 Å, relative intensity 0.500, smoothing width 9999.000, threshold 9999.0.

**Table 4: Lattice plane distances d(A) and intensities obtained for the XPRD-measurement in reference example 1**

| 2-Theta | d(A) | I (counts) |
|---|---|---|
| 6,257 | 14.11403 | 153 |
| 7,145 | 12.36193 | 180 |
| 9,500 | 9.30192 | 74 |
| 10,795 | 8.18875 | 67 |
| 12,586 | 7.02707 | 174 |
| 13,020 | 6.79410 | 136 |
| 14,423 | 6.13620 | 206 |
| 14,916 | 5.93447 | 199 |
| 15,941 | 5.55515 | 337 |
| 16,200 | 5.46681 | 294 |
| 16,889 | 5.24542 | 209 |
| 19,164 | 4.62745 | 459 |
| 21,926 | 4.05046 | 309 |
| 23,840 | 3.72934 | 251 |
| 24,055 | 3.69655 | 312 |
| 25,331 | 3.51317 | 447 |
| 26,128 | 3.40768 | 297 |
| 31, 747 | 2.81627 | 157 |

### Examples 1 to 4

The particles comprising sitagliptin fumarate used in examples 1 - 4 were manufactured using the batch of reference example 1.

Core tablets comprising 100 mg sitagliptin fumarate as dosage strength (example 1; batch no. 1227E006), film-coated tablets (FCT) comprising 25 mg sitagliptin fumarate as dosage strength (example 2; batch no. 1228E003), film-coated tablets comprising 100 mg sitagliptin fumarate as dosage strength (example 3; batch no. 1229E001) and core-tablets comprising 25 µm sitagliptin fumarate as dosage strength (example 4; batch no. 1225E004) were prepared by blending and sieving of the sitaglipitin fumarate together with the excipients, except the lubricants, given in table 5. The lubrication step was made by blending with sodium stearyl fumarate and magnesium stearate. The final blends were compressed on a rotary tablet press to tablets with the compositions given in table 5. For the film-coated tablets, the tablet cores were film-coated in a perforated drum coater with a PVA (polymer) containing ready to use coating e.g. Opadry II-85F23813 (Colorcon) or Aquapolish P rot MR 2284 PVA (Biogrund) according to the amounts given in table 5.

**Table 5: Composition of tablets of Examples 1 - 4**

| **Example** | **Ex. 1** | **Ex. 2** | **Ex. 3** | **Ex.4** |
|---|---|---|---|---|
| Dosage strength and tablet type | 100 mg Core | 25 mg FCT | 100 mg FCT | 25 mg Core |
| Sitagliptin fumarate | 121.3 | 30.3 | 121.3 | 30.3 |
| Corresponding to Sitagliptin | (100) | (25) | (100) | (25) |
| Calcium hydrogen phosphate anhydrous (direct compression grade) | 127.35 | 31.85 | 127.35 | 31.85 |
| Avicel 102 (microcrystalline cellulose) | 127.35 | 31.85 | 127.35 | 31.85 |
| AcDiSol (croscarmellose sodium) | 8.0 | 2.0 | 8.0 | 2.0 |
| Sodiumstearylfumarate (PRUV) | 12.0 | 3.0 | 12.0 | 3.0 |
| Magnesiumstearate | 4.0 | 1.0 | 4.0 | 1.0 |
| Opadry II 85F23813 (Colorcon) | | 4.0 | | |
| Aquapolish P rot MR 2284 PVA (Biogrund) | | | 16 | |
| Sum | 400 | 104 | 416 | 100 |

| | | | | |
|---|---|---|---|---|
| Amounts in Table 3 are given in mg. | | | | |

Some initial properties of the tablets from Examples 1 - 4, obtained using standard procedures (according to European Pharmacopoeia 7.0: 2.9.7 Friability; 2.9.8 Hardness; 2.9.1 Disintegration time; Compression force taken from the tablet maker), can be taken from table 6.

**Table 6: Properties of tablets from Examples 1 - 4**

| **Example** | **Ex. 1** | **Ex. 2** | **Ex. 3** | **Ex.4** |
|---|---|---|---|---|
| Dosage strength and tablet type | 100 mg Core | 25 mg FCT | 100 mg FCT | 25 mg Core |
| Batch size | 1200 g | 811 g | 1091 g | 1000 g |
| Diameter | 10 mm | | | 6 mm |
| Compression force | 5.0 KN | | | 3.2 - 3.7 KN |
| Hardness | 90 - 100 N | | | 76 - 83 N |
| Disintegration time | < 1 Min | < 2 Min | | < 1 Min |
| Friability | 0.1 % | | | 0.17 % |

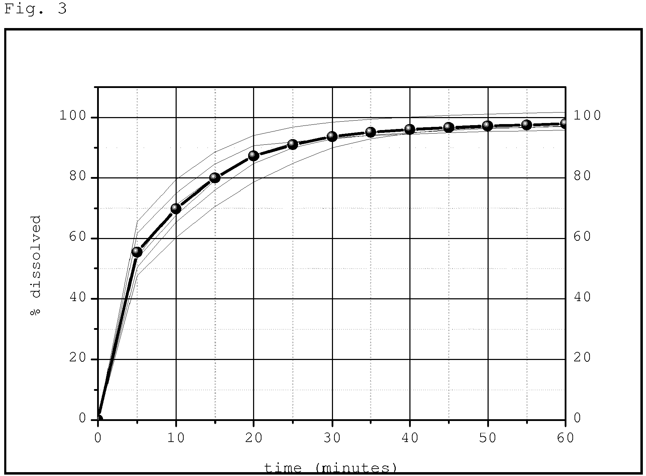

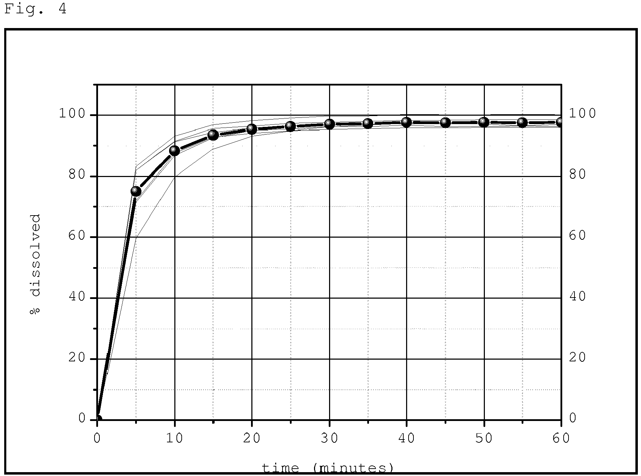

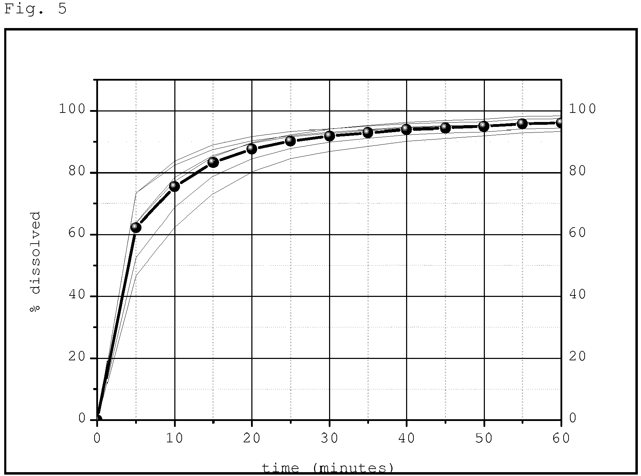

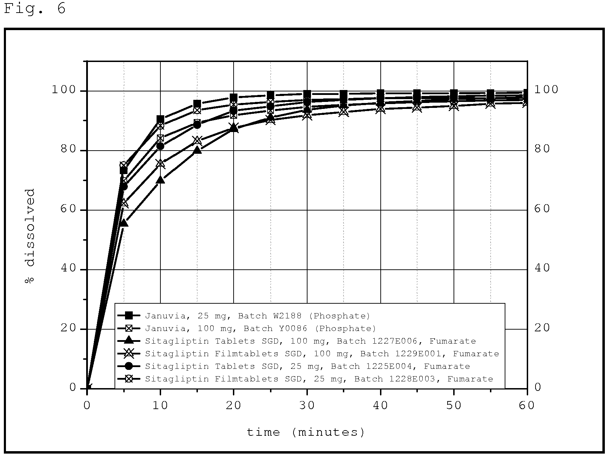

Dissolution experiments were carried out as in comparative examples 1 and 2, and the results for example 1 are given in table 7 and Fig. 3, for example 2 in table 8 and Fig. 4, and for example 3 in table 9 and Fig. 5. Furthermore, a comparison of the dissolution profiles of comparative examples 1 and 2 and examples 1 to 4 is given in Fig. 6.

**Table 7: Dissolution results of core tablets comprising 100 mg sitagliptin fumarate (unmilled) of example 1**

| **Dissolution** | | | | | | | |
|---|---|---|---|---|---|---|---|
| **Time (min.)** | **Tbl. 1 (%)** | **Tbl. 2 (%)** | **Tbl. 3 (%)** | **Tbl. 4 (%)** | **Tbl. 5 (%)** | **Tbl. 6 (%)** | **Mean (%)** |
| 0 | 0.0 | 0.0 | 0.0 | 0.0 | 0.0 | 0.0 | **0.0** |
| 5 | 65.5 | 61.7 | 53.3 | 53.7 | 50.4 | 47.6 | **55.4** |
| 10 | 79.4 | 74.9 | 67.6 | 71.1 | 65.3 | 60.2 | **69.8** |
| 15 | 88.5 | 84.6 | 79.1 | 80.4 | 76.0 | 70.6 | **79.9** |
| 20 | 94.0 | 90.6 | 87.5 | 87.6 | 84.7 | 78.5 | **87.2** |
| 25 | 96.8 | 91.8 | 91.4 | 91.5 | 89.9 | 84.8 | **91.0** |
| 30 | 98.4 | 93.2 | 93.6 | 93.6 | 92.8 | 89.9 | **93.6** |
| 35 | 99.4 | 94.0 | 95.1 | 94.9 | 94.1 | 92.9 | **95.1** |
| 40 | 100.1 | 94.4 | 95.9 | 95.6 | 94.9 | 94.8 | **96.0** |
| 45 | 100.7 | 94.9 | 96.5 | 96.2 | 95.7 | 95.9 | **96.6** |
| 50 | 101.1 | 95.3 | 97.1 | 96.5 | 96.3 | 96.8 | **97.2** |
| 55 | 101.4 | 95.5 | 97.4 | 96.7 | 96.7 | 97.4 | **97.5** |
| 60 | 101.7 | 95.8 | 97.7 | 97.0 | 97.1 | 97.9 | **97.9** |

**Table 8: Dissolution results of film coated tablets comprising 25 mg sitagliptin fumarate (unmilled) of example 2**

| **Dissolution** | | | | | | | |
|---|---|---|---|---|---|---|---|
| **Time (min.)** | **Tbl. 1 (%)** | **Tbl. 2 (%)** | **Tbl. 3 (%)** | **Tbl. 4 (%)** | **Tbl. 5 (%)** | **Tbl. 6 (%)** | **Mean (%)** |
| 0 | 0.0 | 0.0 | 0.0 | 0.0 | 0.0 | 0.0 | **0.0** |
| 5 | 72.0 | 71.4 | 81.9 | 81.9 | 59.6 | 83.2 | **75.0** |
| 10 | 87.5 | 86.8 | 91.5 | 91.3 | 79.7 | 93.2 | **88.3** |
| 15 | 92.8 | 92.6 | 95.7 | 94.4 | 88.9 | 96.9 | **93.5** |
| 20 | 94.1 | 95.0 | 96.4 | 95.8 | 93.2 | 98.2 | **95.4** |
| 25 | 94.9 | 95.5 | 97.3 | 96.5 | 94.8 | 99.1 | **96.3** |
| 30 | 95.3 | 96.3 | 97.7 | 96.8 | 96.2 | 99.7 | **97.0** |
| 35 | 95.6 | 96.2 | 98.0 | 97.0 | 96.8 | 99.7 | **97.2** |
| 40 | 95.8 | 96.7 | 98.3 | 97.1 | 97.3 | 100.3 | **97.6** |
| 45 | 95.8 | 96.2 | 98.3 | 97.1 | 97.3 | 100.2 | **97.5** |
| 50 | 95.9 | 96.3 | 98.5 | 97.1 | 97.5 | 100.2 | **97.6** |
| 55 | 95.9 | 96.4 | 98.5 | 96.4 | 97.7 | 100.3 | **97.5** |
| 60 | 96.0 | 96.3 | 98.5 | 97.0 | 97.7 | 100.2 | **97.6** |

**Table 9: Dissolution results of film coated tablets comprising 100 mg sitagliptin fumarate (unmilled) of example 3**

| **Dissolution** | | | | | | | |
|---|---|---|---|---|---|---|---|
| **Time (min.)** | **Tbl. 1 (%)** | **Tbl. 2 (%)** | **Tbl. 3 (%)** | **Tbl. 4 (%)** | **Tbl. 5 (%)** | **Tbl. 6 (%)** | **Mean (%)** |
| 0 | 0.0 | 0.0 | 0.0 | 0.0 | 0.0 | 0.0 | **0.0** |
| 5 | 63.8 | 73.3 | 73.4 | 52.4 | 46.6 | 63.7 | **62.2** |
| 10 | 78.5 | 83.7 | 82.4 | 68.7 | 62.3 | 77.2 | **75.5** |
| 15 | 85.5 | 89.0 | 87.4 | 78.9 | 73.2 | 85.0 | **83.2** |
| 20 | 89.5 | 91.7 | 90.3 | 84.4 | 80.2 | 89.7 | **87.6** |
| 25 | 91.6 | 93.3 | 92.0 | 87.8 | 84.5 | 92.2 | **90.2** |
| 30 | 92.7 | 94.1 | 93.1 | 89.8 | 86.8 | 94.0 | **91.8** |
| 35 | 93.6 | 95.0 | 93.9 | 91.1 | 88.5 | 95.2 | **92.9** |
| 40 | 94.4 | 95.7 | 94.6 | 92.2 | 90.1 | 96.2 | **93.9** |
| 45 | 94.9 | 96.1 | 95.1 | 92.8 | 91.0 | 96.8 | **94.4** |
| 50 | 95.2 | 96.4 | 95.3 | 93.2 | 91.9 | 97.2 | **94.9** |
| 55 | 96.0 | 97.2 | 96.1 | 94.1 | 92.9 | 98.1 | **95.7** |
| 60 | 96.2 | 97.4 | 96.3 | 94.3 | 93.3 | 98.3 | **96.0** |

### Comparative examples 3 and 4

A film coated tablet was manufactured in the same way as in example 2, except that sitagliptin fumarate batches with a different particle size were used, wherein the sitagliptin fumarate particles were milled. The particle sizes of the sitagliptin fumarate used in comparative examples 3 and 4 were measured using sieve analysis as described before in Reference Example 1 with sieves with mesh sizes of 63 µm, 125 µm and 250 µm. As a result the particles in comparative example 3 were all smaller than 63 µm (batch no. 1205E007) and in comparative example 4 all smaller than 125 µm (batch no. 1205E008).

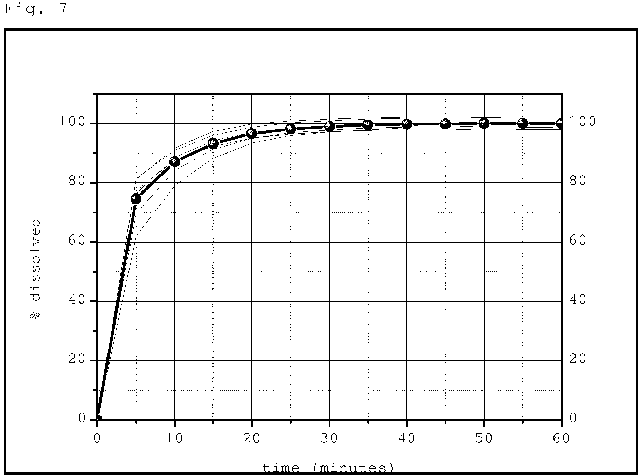

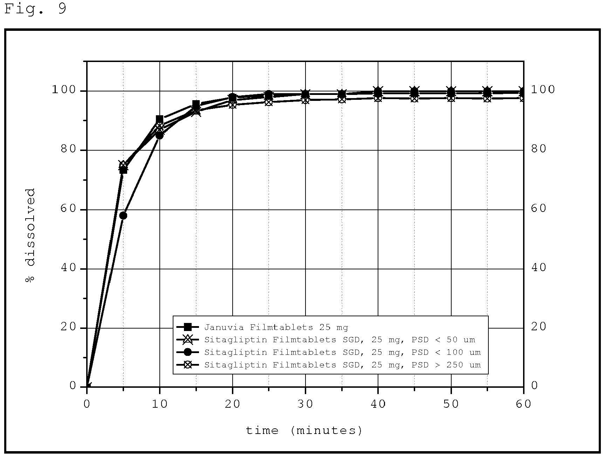

The results of dissolution tests of the film coated tablets, carried out in the same way as in example 2, are given in Figs. 7 (comparative example 3) and 8 (comparative example 4). For comparison, the results of the dissolution tests of comparative examples 1, 3 and 4 and example 2 are given in Fig. 9. Further, for additional comparison, the average dissolution results of comparative examples 1 and 2, examples 1 - 3 and comparative examples 3 and 4 are given in the following tables 10 to 16

**Table 10: Average dissolution results of comparative example 1**

| Time (min.) | 0 | 5 | 10 | 15 | 20 | 25 | 30 | 35 | 40 | 45 | 50 | 55 | 60 |
|---|---|---|---|---|---|---|---|---|---|---|---|---|---|
| Mean (%) | 0 | 71 | 88 | 93 | 95 | 96 | 96 | 96 | 96 | 96 | 96 | 96 | 96 |

**Table 11: Average dissolution results of comparative example 2**

| Time (min.) | 0 | 5 | 10 | 15 | 20 | 25 | 30 | 35 | 40 | 45 | 50 | 55 | 60 |
|---|---|---|---|---|---|---|---|---|---|---|---|---|---|
| Mean (%) | **0** | **70** | **84** | **89** | **92** | **93** | **95** | **95** | **96** | **96** | **97** | **97** | **97** |

**Table 12: Average dissolution results of example 1**

| Time (min.) | 0 | 5 | 10 | 15 | 20 | 25 | 30 | 35 | 40 | 45 | 50 | 55 | 60 |
|---|---|---|---|---|---|---|---|---|---|---|---|---|---|
| Mean (%) | **0** | **55** | **70** | **80** | **87** | **91** | **94** | **95** | **96** | **97** | **97** | **98** | **98** |

**Table 13: Average dissolution results of example 2**

| Time (min.) | 0 | 5 | 10 | 15 | 20 | 25 | 30 | 35 | 40 | 45 | 50 | 55 | 60 |
|---|---|---|---|---|---|---|---|---|---|---|---|---|---|
| Mean (%) | **0** | **75** | **88** | **94** | **95** | **96** | **97** | **97** | **98** | **98** | **98** | **98** | **98** |

**Table 14: Average dissolution results of example 3**

| Time (min.) | 0 | 5 | 10 | 15 | 20 | 25 | 30 | 35 | 40 | 45 | 50 | 55 | 60 |
|---|---|---|---|---|---|---|---|---|---|---|---|---|---|
| Mean (%) | **0** | **62** | **76** | **83** | **88** | **90** | **92** | **93** | **94** | **94** | **95** | **96** | **96** |

**Table 15: Average dissolution results of comparative example 3**

| Time (min.) | 0 | 5 | 10 | 15 | 20 | 25 | 30 | 35 | 40 | 45 | 50 | 55 | 60 |
|---|---|---|---|---|---|---|---|---|---|---|---|---|---|
| Mean (%) | 0 | 75 | 87 | 93 | 97 | 98 | 99 | 99 | 100 | 100 | 100 | 100 | 100 |

**Table 16: Average dissolution results of comparative example 4**

| Time (min.) | 0 | 5 | 10 | 15 | 20 | 25 | 30 | 35 | 40 | 45 | 50 | 55 | 60 |
|---|---|---|---|---|---|---|---|---|---|---|---|---|---|
| Mean (%) | 0 | 58 | 85 | 95 | 98 | 99 | 99 | 99 | 100 | 100 | 100 | 100 | 100 |

As can be seen from Figs. 6 and 9 and tables 2, 3 and 7 to 16 the dissolution characteristics of the examples are comparable to the ones of the comparative examples, showing that the pharmaceutical formulations of the present invention can be used in a similar manner as already marketed compounds as well as formulations using particle mixtures with particle sizes substantially smaller regarding than the present ones, while they can be more easily manufactured by direct compression without further pretreatment and show improved stability. In this respect it is surprising that tablets comprising the present pharmaceutical formulations wherein at least 45 wt.% of the active pharmaceutical ingredient (API) have a size of more than 250 µm nevertheless can be dissolved in the same time as tablets of formulations wherein the API is present in the form of much smaller particles, as in e.g. comparative examples 3 and 4.

The present pharmaceutical formulation comprising DPP-IV inhibitor particles comprising DPP-IV inhibitor agglomerates and DPP-IV inhibitor particles, wherein the DPP-IV inhibitor is in free form or in form of a salt thereof, wherein at least 45 wt.% of the DPP-IV inhibitor particles and DPP-IV inhibitor agglomerates have a size when measured using sieve analysis of more than 250 µm allows the production of solid oral dosage forms, particularly tablets and coated tablets, with improved stability, wherein the production is simplified due to the good flow properties of the agglomerates of the DPP-IV inhibitor particles. Solid oral dosage forms, particularly tablets and coated tablets, can be easily produced in a reproducible manner with good hardness and dissolution characteristics due to the good flow properties.

## Claims

1. A pharmaceutical formulation comprising DPP-IV inhibitor agglomerates and DPP-IV inhibitor particles, wherein the DPP-IV inhibitor is in free form or in form of a salt thereof, wherein at least 45 wt.% of the DPP-IV inhibitor particles and DPP-IV inhibitor agglomerates have a size when measured using sieve analysis of more than 250 µm.

2. The pharmaceutical formulation of claim 1, wherein the DPP-IV inhibitor is (R)-4-oxo-4-[3-(trifluoromethyl)-5,6-dihydro[1,2,4]triazolo[4,3-a]pyrazin-7(8H)-yl]-1-(2,4,5-trifluorophenyl)butan-2-amine (sitagliptin) or a salt thereof.

3. The pharmaceutical formulation of claim 1 or 2, wherein the DPP-IV inhibitor is the fumaric acid salt of (R)-4-oxo-4-[3-(trifluoromethyl)-5,6-dihydro[1,2,4]triazolo[4,3-a]pyrazin-7(8H)-yl]-1-(2,4,5-trifluorophenyl)butan-2-amine.

4. The pharmaceutical formulation of any one of the preceding claims, further comprising one or more pharmaceutically acceptable excipient.

5. The pharmaceutical formulation of any one of the preceding claims, further comprising a second pharmaceutically active ingredient.

6. The pharmaceutical formulation of claim 5, wherein the second pharmaceutically active ingredient is a biguanide, preferably metformin, more preferably metformin hydrochloride.

7. The pharmaceutical formulation of any one of the preceding claims, wherein the formulation is in the form of an oral dosage form.

8. The pharmaceutical formulation of any one of the preceding claims, wherein the formulation is in the form of a tablet, coated tablet, capsule, pill, powder or granules.

9. The pharmaceutical formulation of any one of the preceding claims for use in the treatment or prevention of non-insulin dependent diabetes mellitus, obesity, insulin resistance, syndrome X and type 2 diabetes.

10. The pharmaceutical formulation of any one of the preceding claims, wherein the DPP-IV inhibitor is a polymorph of the fumaric acid salt of (R)-4-oxo-4-[3-(trifluoromethyl)-5,6-dihydro[1,2,4]triazolo[4,3-a]pyrazin-7(8H)-yl]-1-(2,4,5-trifluorophenyl)butan-2-amine which exhibits a characteristic X-ray powder diffraction pattern with the following characteristic peaks of the following 2-theta values when measured using Kα-radiation of a copper anode:
| 2-theta | I (counts) |
|---|---|
| 6,257 | 153; |
| 7,145 | 180; |
| 9,500 | 74; |
| 10,795 | 67; |
| 12,586 | 174; |
| 13,020 | 136; |
| 14,423 | 206; |
| 14,916 | 199; |
| 15,941 | 337; |
| 16,200 | 294; |
| 16,889 | 209; |
| 19,164 | 459; |
| 21,926 | 309; |
| 23,840 | 251; |
| 24,055 | 312; |
| 25,331 | 447; |
| 26,128 | 297; |
| 31,747 | 157. |

11. A DPP-IV inhibitor agglomerate comprising a DPP-IV inhibitor in free form or in form of a salt thereof, wherein the DPP-IV inhibitor agglomerate has a size in the range of about 250 µm - about 1000 µm.

12. The DPP-IV inhibitor agglomerate of claim 11, wherein the DPP-IV inhibitor is (R)-4-oxo-4-[3-(trifluoromethyl)-5,6-dihydro[1,2,4]triazolo[4,3-a]pyrazin-7(8H)-yl]-1-(2,4,5-trifluorophenyl)butan-2-amine or a salt thereof.

13. The DPP-IV inhibitor agglomerate of claim 11 or 12, wherein the DPP-IV inhibitor is the fumaric acid salt of (R)-4-oxo-4-[3-(trifluoromethyl)-5,6-dihydro[1,2,4]triazolo[4,3-a]pyrazin-7(8H)-yl]-1-(2,4,5-trifluorophenyl)butan-2-amine.

14. The DDP-IV inhibitor agglomerate of any one of claims 11 to 13, wherein the DPP-IV inhibitor is a polymorph of the fumaric acid salt of (R)-4-oxo-4-[3-(trifluoromethyl)-5,6-dihydro[1,2,4]triazolo[4,3-a]pyrazin-7(8H)-yl]-1-(2,4,5-trifluorophenyl)butan-2-amine which exhibits a characteristic X-ray powder diffraction pattern with the following characteristic peaks of the following 2-theta values when measured using Kα-radiation of a copper anode:
| 2-theta | I (counts) |
|---|---|
| 6,257 | 153; |
| 7,145 | 180; |
| 9,500 | 74; |
| 10,795 | 67; |
| 12,586 | 174; |
| 13,020 | 136; |
| 14,423 | 206; |
| 14,916 | 199; |
| 15,941 | 337; |
| 16,200 | 294; |
| 16,889 | 209; |
| 19,164 | 459; |
| 21,926 | 309; |
| 23,840 | 251; |
| 24,055 | 312; |
| 25,331 | 447; |
| 26,128 | 297; |
| 31,747 | 157. |
